# EUROPÄISCHE PATENTANMELDUNG

(11) **EP 1 114 627 A1**
(43) Veröffentlichungstag der Anmeldung: **11.07.2001**
(21) Anmeldenummer: 00811137.9
(22) Anmeldetag: 30.11.2000
(51) Int. Cl.: A61F 9/007

(54) **Verfahren und Vorrichtung zum Verbessern des Kammerwasserabflusses in einem Auge.**

(30) Priorität: 05.01.2000 US 478047
(71) Anmelder: GRIESHABER & CO. AG SCHAFFHAUSEN, CH-8203 Schaffhausen (CH)
(72) Erfinder: Grieshaber, Hans R., 8200 Schauffhausen (CH); Stegmann, Robert, Prof. M.D., Pretoria 0181 (ZA)
(74) Vertreter: Althoff, Gerhard

(57) **Zusammenfassung**

Es wird ein Verfahren zum Verbessern des Kammerwasserabflusses in einem Auge sowie eine Vorrichtung zum Aufweiten des schlemmschen Kanals vorgeschlagen.

Gemäss dem Verfahren wird durch einen ersten Einschnitt und einem innerhalb des ersten Einschnitts angeordneten zweiten Einschnitt (Inzision) an der Oberfläche der Sklera (3) und durch jeweiligem Aufklappen der analog der Einschnitte ausgebildeten skleralappen (10) und (12) der Schlemmsche Kanal (5) zum Injizieren eines hochviskosen Medium im Bereich eines Teilstücks (18) freigelegt. Zur Bildung eines die kammerwasserdurchlässige Descemet-Membrane (6) mit einem subskleralen Raum (13') verbindenden Spalts (21) wird mit geringer Anpresskraft im Bereich der Schwalbeschen Linie (7) die Descemet-Membrane (6) von der Kornea (4) gelöst. Anschliessend wird der zweite Skleralappen (12) abgeschnitten und der erste Skleralappen (10) auf eine Auflagefläche (14) gelegt und der subsklerale Raum (13') vor dem vollständigen Vernähen mit hochviskosem Medium gefüllt.

Die Vorrichtung zum Dehnen des chirurgisch freigelegten Schlemmschen Kanals (5) umfasst eine in den Schlemmschen Kanal (5) einführbare und für das zu injizierende Medium mit mindestens einem Austrittskanal versehene Sonde, welche zum Injizieren des hochviskosen Mediums über ein Anschlussteil und eine Zuführleitung mit einer Druckquelle in Wirkverbindung steht.

## Beschreibung

Die Erfindung bezieht sich auf ein Verfahren zum Verbessern des Kammerwasserabflusses in einem Auge sowie auf eine Vorrichtung zum Aufweiten des zirkulären Schlemmschen Kanals.

Aus den Druckschriften (US-A 5,360,399 und US-A 5,486,165) ist ein Verfahren sowie eine Vorrichtung zur Durchführung des Verfahrens bekannt, mittels welchem/welcher das dem Schlemmschen Kanal vorgelagerte und infolge krankhafter Veränderungen den Abfluss des Kammerwassers teilweise oder vollständig obstruierende Trabekulargewebe mittels einer in den Schlemmschen Kanal injizierten hochviskosen, wässrigen Lösung im wesentlichen hydraulisch gedehnt wird, und dadurch an einigen Stellen für den Abfluss des Kammerwassers geöffnet wird.

Der Erfindung liegt die Aufgabe zugrunde ein Verfahren anzugeben sowie eine Vorrichtung zu schaffen, mittels welchem/welcher der erforderliche Abfluss des Kammerwassers über das natürliche Kanalsystem in einem Auge und infolge dessen eine den Druck regulierende Zirkulation des Kammerwassers verbessert wird.

Die Aufgabe hinsichtlich des Verfahrens ist gekennzeichnet durch einen ersten lamellaren Einschnitt an der Oberfläche der Sklera mit in Richtung der Kornea aufgeschwenktem ersten Skleralappen und analog ausgebildeter erster Ausnehmung, einen innerhalb der ersten Ausnehmung angeordneten zweiten lamellaren Einschnitt mit in Richtung des ersten Skleralappens aufgeschwenktem zweiten Skleralappen und analog ausgebildeter zweiter Ausnehmung sowie ein im Bereich der zweiten Ausnehmung freigelegtes Teilstück des Schlemmschen Kanals mit zwei gegenüberliegenden Öffnungen zum Injizieren eines den Schlemmschen Kanal dehnenden Mediums, wobei der erste Skleralappen nach dem Abtrennen des zweiten Skleralappens auf eine analog dem zweiten Einschnitt ausgebildete Auflagefläche gelegt wird und der dadurch gebildete subsklerale Raum vor dem vollständigen Verschliessen mit einem viskosen Medium gefüllt wird.

Nach einem weiteren Merkmal des Verfahrens besteht zudem die Möglichkeit, dass zur Bildung eines die kammerwasserdurchlässige Descemet-Membran mit der zweiten Ausnehmung beziehungsweise mit dem subskleralen Raum verbindenden Spalts die Descemet-Membrane durch eine geringe Anpresskraft im Bereich der Schwalbeschen Linie von der Kornea gelöst wird.

Hierdurch wird erreicht, dass das in natürlicherweise über das Trabekulargewebe in den Schlemmschen Kanal abgeleitete Kammerwasser zusätzlich noch von der Vorderkammer über die Descemt-Membran und den Spalt in den mit dem Schlemmschen Kanal in Verbindung stehenden subskleralen Raum gelangen kann.

Die Vorrichtung ist gekennzeichnet durch eine in den Schlemmschen Kanal einführbare und für das zu injizierende Medium mit mindestens einem Austrittskanal versehene Sonde, welche eine in axialer Richtung orientierte und mindestens dem zweifachen Durchmesser entsprechende Länge aufweist.

Weitere Vorteile, Merkmale und Einzelheiten der Erfindung ergeben sich aus den einzelnen Patentansprüchen, der nachstehenden Beschreibung und der Zeichnung.

Die Erfindung wird nachstehend anhand von Ausführungsbeispielen in Verbindung mit der Zeichnung beschrieben. Es zeigt:
- **Fig.1**: ein in schematischer Ansicht und in grösserem Massstab dargestelltes Teilstück eines Auges mit einem ersten parabolischen Einschnitt in der Sklera und aufgeklapptem ersten Skleralappen;
- **Fig.2**: das entlang der Linie II-II in Fig.1 im Schnitt dargestellte Teilstück des Auges mit aufgeklapptem ersten Skleralappen;
- **Fig.3**: das Teilstück des Auges gemäss Fig.1 mit einem innerhalb des ersten parabolischen Einschnitts angeordneten zweiten parabolischen Einschnitt und aufgeklapptem zweiten Skleralappen;
- **Fig.4**: das entlang der Linie IV-IV in Fig.3 im Schnitt dargestellte Teilstück des Auges mit den beiden aufgeklappten Skleralappen;
- **Fig.5**: ein erstes Ausführungsbeispiel einer in den freigelegten Schlemmschen Kanal eingeführten und an einem bogenförmigen Anschlussteil angeordneten Sonde;
- **Fig.6**: ein zweites Ausführungsbeispiel der in den freigelegten Schlemmschen Kanal eingeführten und an einem T-förmigen Anschlussteil angeordneten Sonde;
- **Fig.7**: das Teilstück des Auges gemäss Fig.4 mit einem im Bereich der beiden aufgeklappten Skleralappen mit geringer Kraft im wesentlichen gegen die Schwalbesche Linie wirkenden Tupfer;
- **Fig. 8**: das Teilstück des Auges gemäss Fig.7 bei welchem die Descemets-Membran von der Kornea gelöst und der zweite Skleralappen abgetrennt ist;
- **Fig.9**: das Teilstück des Auges gemäss Fig.8 mit heruntergeklapptem ersten Skleralappen;
- **Fig.10**: ein in Ansicht sowie in grösserem Massstab dargestelltes erstes Ausführungsbeispiel der an einem Anschlussteil angeformten Sonde;
- **Fig.10A**: eine erste Variante der an dem Anschlussteil gemäss Fig.10 angeordneten Sonde;
- **Fig.10B**: eine zweite Variante der an dem Anschlussteil gemäss Fig.10 angeordneten Sonde;
- **Fig.11**: ein in Ansicht und teilweise im Schnitt dargestelltes zweites Ausführungsbeispiel der an einem Anschlussteil angeformten Sonde;
- **Fig.11A**: das entlang der Linie XI-XI im Schnitt dargestellte Anschlussteil gemäss Fig.11 mit der im Profilquerschnitt dargestellten Sonde;
- **Fig.11B**: das Anschlussteil gemäss Fig.11 mit einer ersten Variante der angeformten Sonde;
- **Fig.11C**: eine im Profilquerschnitt dargestellte zweite Variante der Sonde gemäss Fig.11B;
- **Fig.11D**: eine im Profilquerschnitt dargestellte dritte Variante der Sonde gemäss Fig.11B;
- **Fig.12**: ein in Ansicht und teilweise im Schnitt dargestelltes drittes Ausführungsbeispiel der an einem bogenförmigen Anschlussteil angeformten Sonde;
- **Fig.12A**: das entlang der Linie XII-XII im Schnitt dargestellte Anschlussteil gemäss Fig.12 mit der im Profilquerschnitt dargestellten Sonde;
- **Fig.12B**: eine im Profilquerschnitt dargestellten Variante der Sonde gemäss Fig.12A; und
- **Fig.13**: das T-förmige Anschlussteil der Injektionseinheit gemäss Fig.6 mit zwei gegenüberliegend angeformten Sonden.

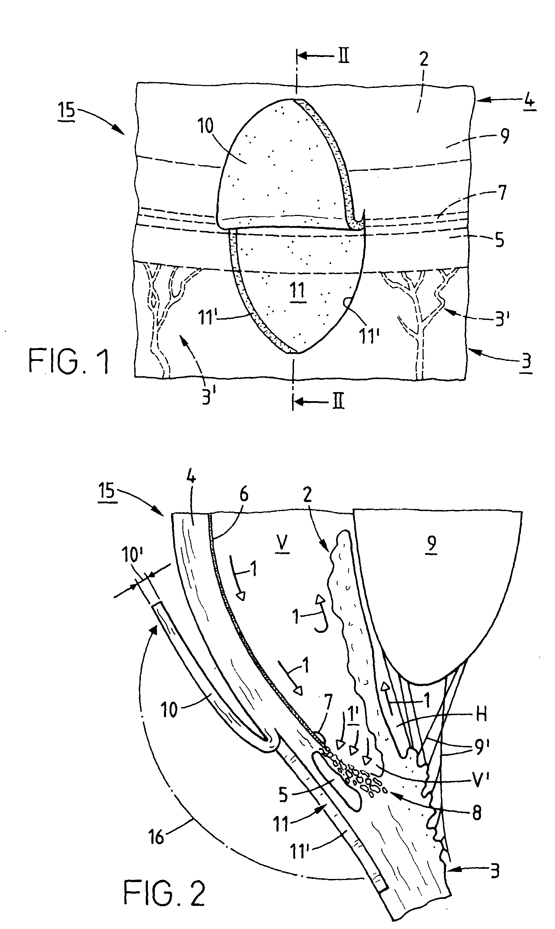

Fig.1 zeigt ein in grösserem Massstab sowie in schematischer Ansicht dargestelltes Teilstück eines Auges 15 und man erkennt jeweils ein Teilstück der Regenbogenhaut 2 (Iris), der Hornhaut 4 (Kornea), der Lederhaut 3 (Sklera), ein Teilstück des zirkulären Schlemmschen Kanals 5 (Sinus venosus sclera) und das aus einer Vielzahl von Kanälen gebildete Kanalsystem 3' für das Kammerwasser. Weiterhin erkennt man in Fig.1 einen ersten etwa parabolisch ausgebildeten Einschnitt (Inzision) in der Sklera 3, einen analog dem Einschnitt ausgebildeten und in Richtung der Kornea 4 aufgeschwenkten Skleralappen 10 sowie eine analog demselben ausgebildete erste Ausnehmung 11 mit einer umlaufenden Seitenwand 11'. Der Skleralappen 10 wird durch nicht dargestellte Mittel in der aufgeschwenkten Position gehalten.

In Fig.2 ist das Teilstück des Auges 15 gemäss der in Fig.1 eingezeichneten Linie II-II im Schnitt sowie in grösserem Massstab dargestellt und man erkennt ein Teilstück der Sklera 3, ein Teilstück der Kornea 4 mit der Descemet-Membrane 6 (Descemet's Membran) und der Schwalbeschen Linie 7 (Schwalbe's line), ein Teilstück der Regenbogenhaut 2 sowie ein Teilstück der mittels der Zonularfasern 9' mit der Lederhaut 3 verbundenen Linse 9. Weiterhin erkennt man den gemäss Pfeilrichtung 16 aufgeschwenkten ersten Skleralappen 10 mit der analog ausgebildeten ersten Ausnehmung 11 sowie den Schlemmschen Kanal 5 mit dem vorgelagerten Trabekulargewebe 8 (trabecular meshwork).

In Fig.2 ist mit den Pfeilen 1 im wesentlichen die Zirkulation des Kammerwassers (humor aquosus) und mit den Pfeilen 1' der natürliche Abfluss dargestellt. Bei einem gesunden Auge zirkuliert das sich ständig erneuernde Kammerwasser gemäss Pfeilrichtung 1 von der Hinterkammer H zur Vorderkammer V und wird im Kammerwinkel V' (angulus irido-cornealis) gemäss der Pfeile 1' über das Trabekulargewebe 8 in den Schlemmschen Kanal 5 und von dort über das Kanalsystem 3' (Fig.1) in das nicht dargestellte natürliche Venensystem abgeleitet. Bei einem infolge Verstopfung oder dergleichen nur teilweise funktionsfähigen beziehungsweise funktionsunfähigen Trabekulargewebe 8 kann der natürliche Abfluss des Kammerwassers derart begrenzt werden, dass der Druck im Inneren des Auges 15 ansteigt und infolge dessen die Durchblutung und somit die Funktion der Sehnerven (nicht dargestellt) eingeschränkt wird. Diese Krankheit ist allgemein unter dem Begriff "Glaukom" bekannt, welche zur Erblindung des betroffenen Auges führen kann.

Für den mikrochirurgischen Eingriff wird zunächst die nicht dargestellte Bindehaut des Auges mit geeigneten Mitteln zurückgezogen und dadurch ein ausreichendes Teilstück der Sklera 3 für den ersten parabolförmigen Einschnitt (Inzision) freigelegt. Nach dem ersten Einschnitt wird der dem Einschnitt entsprechend ausgebildete erste Skleralappen 10 in Richtung der Kornea 4 geklappt und infolge dessen die erste Ausnehmung 11 mit der umlaufenden Seitenwand 11' freigelegt. Die Tiefe des ersten beispielsweise 3mm x 3mm grossen Einschnitts ist beispielsweise so gewählt, dass die Dicke 10' des in Fig.2 im Profilquerschnitt dargestellten ersten Skleralappens 10 etwa 1/3 der in diesem Bereich natürlichen Dicke der Sklera 3 entspricht. Der Schlemmsche Kanal 5 ist in dieser ersten Phase (Fig.2) noch nicht freigelegt.

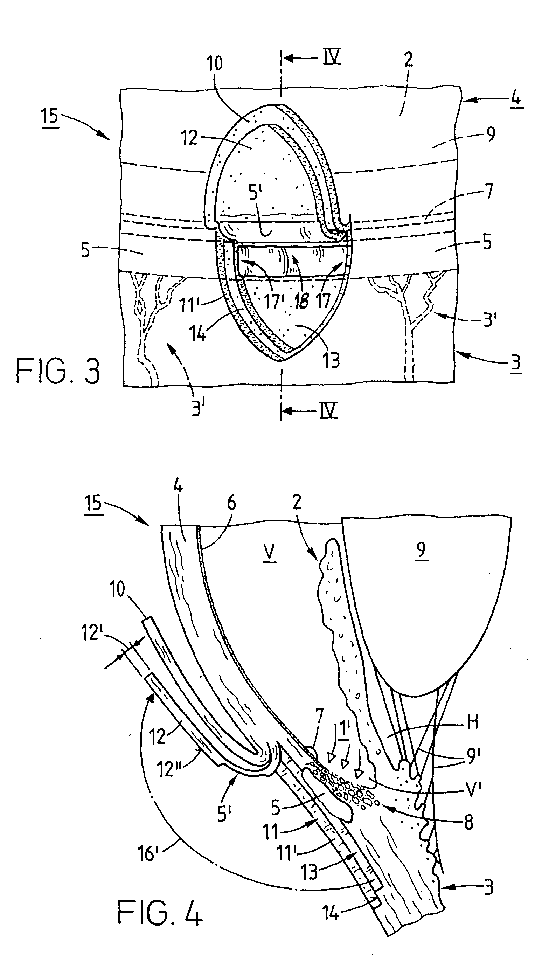

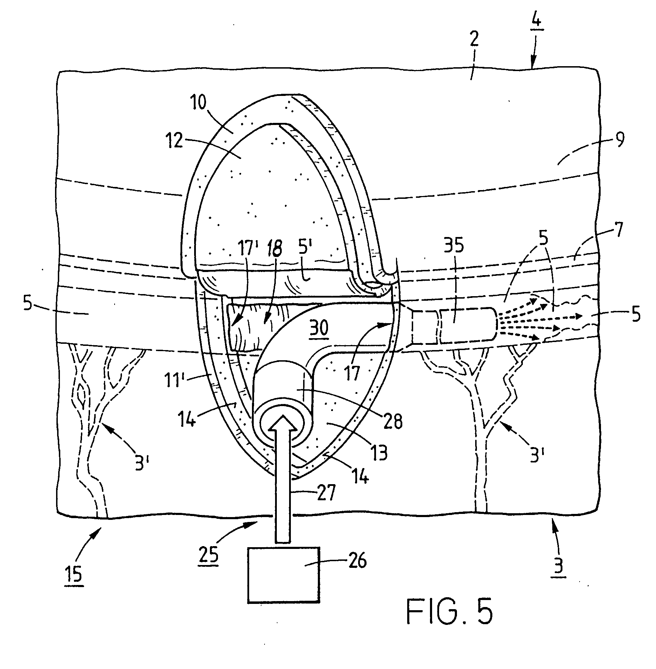

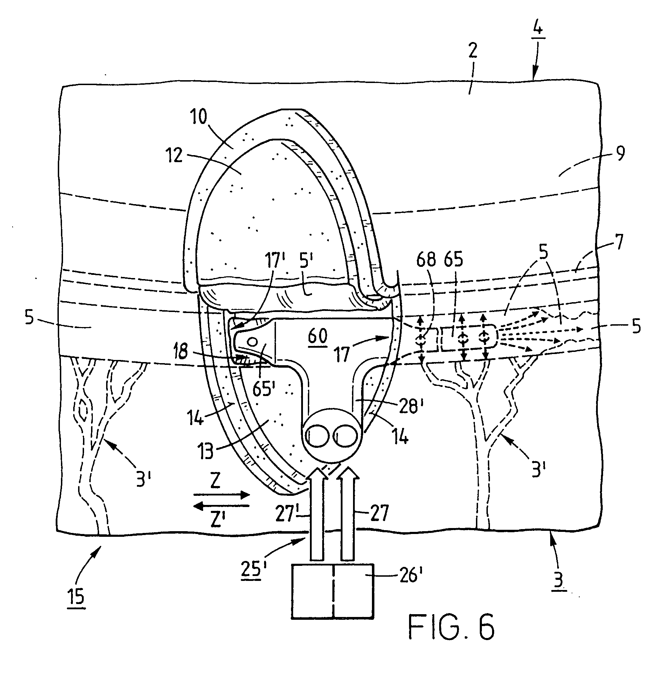

Fig.3 zeigt das Teilstück des Auges 15 mit einem innerhalb des ersten parabolischen Einschnitts angeordneten zweiten Einschnitt. Nach der Durchführung des zweiten Einschnitts wird ein entsprechend parabolförmig ausgebildeter zweiter Skleralappen 12 in Richtung der Kornea 4 aufgeklappt, infolge dessen eine analog des zweiten Skleralappens 12 ausgebildete zweite Ausnehmung 13 freigelegt wird. Die Tiefe des zweiten Einschnitts ist so gewählt, dass in dieser Phase der Schlemmsche Kanal 5 bei aufgeschwenktem zweiten Skleralappen 12 über die gesamte Breite des mit 18 bezeichneten Teilstücks der Ausnehmung 13 freigelegt ist. In dieser Phase sind die beiden im Bereich des Teilstücks 18 gegenüberliegend zueinander angeordneten Öffnungen 17 und 17' des Schlemmschen Kanals 5 zum Einführen einer entsprechend ausgebildeten Sonde (Fig.5 und Fig.6) zugänglich.

In Fig.4 ist das Teilstück des Auges 15 gemäss der in Fig.3 eingezeichneten Linie IV-IV mit den beiden gemäss Pfeilrichtung 16 (Fig.3) und Pfeilrichtung 16' (Fig.4) aufgeschwenkten Skleralappen 10 und 12 im Schnitt dargestellt. Die beiden Skleralappen 10 und 12 sind für den weiteren Eingriff mit nicht dargestellten Mitteln in dieser Position gehalten. Die Schnitttiefe sowie die davon abhängige Dicke 12' des in Fig.4 im Profilquerschnitt dargestellten zweiten Skleralappens 12 ist beispielsweise so gewählt, dass der Schlemmsche Kanal 5 gut zugänglich freigelegt ist. Dies wird im wesentlichen auch dadurch erreicht, dass infolge der gewählten Schnitttiefe an der Innenseite 12" des zweiten Skleralappens 12 noch ein Teilstück 5' des Schlemmschen Kanals 5 vorhanden ist. In Fig.3 ist das in Form einer sich über die gesamte Breite des zweiten Skleralappens 12 erstreckende etwa rillenförmige
Teilstück 5' des Schlemmschen Kanals 5 dargestellt. Weiterhin erkennt man in Fig.3 und Fig.4 die beiden Ausnehmungen 11 und 13 mit der Seitenwand 11' und der Auflagefläche 14.

In einer dritten Phase wird mittels einer entsprechend ausgebildeten Injektionseinheit 25 oder 25' gemäss Fig.5 oder Fig.6 in die beiden seitlichen Öffnungen 17 und 17' des freigelegten Schlemmschen Kanals 5 ein geeignetes Medium, vorzugsweise eine hochviscose Natrium-Hyaluronat-Lösung (high viscosity hyaluronate) injiziert. Hierdurch wird das Lumen des Schlemmschen Kanals 5 mindestens über die gesamte Länge der eingeführten Sonde aufgeweitet.

In Fig.5 ist das Teilstück des Auges 15 gemäss Fig.3 in grösserem Massstab dargestellt und man erkennt ein Teilstück der Sklera 3 sowie die beiden in Richtung der Kornea 4 aufgeschwenkten beziehungsweise hochgeklappten Skleralappen 10 und 12, die zweite Ausnehmung 13 mit der seitlichen Auflagefläche 14 der Sklera 3 sowie das freigelegte Teilstück 18 des Schlemmschen Kanals 5. Zum Injizieren der hochviscosen Natrium-Hyaluronat-Lösung ist in Fig.5 weiterhin eine erste Injektionseinheit 25 dargestellt, welche mit einer an einem bogenförmigen Anschlussteil 30 angeordneten ersten Sonde 35 in die freigelegte Öffnung 17 des Schlemmschen Kanals 5 eingeführt ist. Nach dem Aufweiten des Lumens mittels der injizierten Natrium-Hyaluronat-Lösung auf der einen Seite wird die Injektionseinheit 25 mit der Sonde 35 aus der Öffnung 17 herausgezogen und in nicht näher dargestellter Weise entsprechend gedreht in die andere, gegenüberliegende Öffnung 17' des Schlemmschen Kanals 5 zum Injizieren des Mediums beziehungsweise zum Aufweiten des Lumens eingeführt.

Die in Fig.5 dargestellte Injektionseinheit 25 steht über eine daran angeschlossene Zuführleitung 28 mit einer schematisch dargestellten Druckquelle 26 in Form einer Einkammer-Spritze oder dergleichen in Verbindung. Mittels der beispielsweise manuell oder elektrisch betätigbaren Druckquelle 26 wird das zu injizierende Medium gemäss Pfeilrichtung 27 in das Lumen des Schlemmschen Kanals 5 gepresst und dieser entsprechend gedehnt.

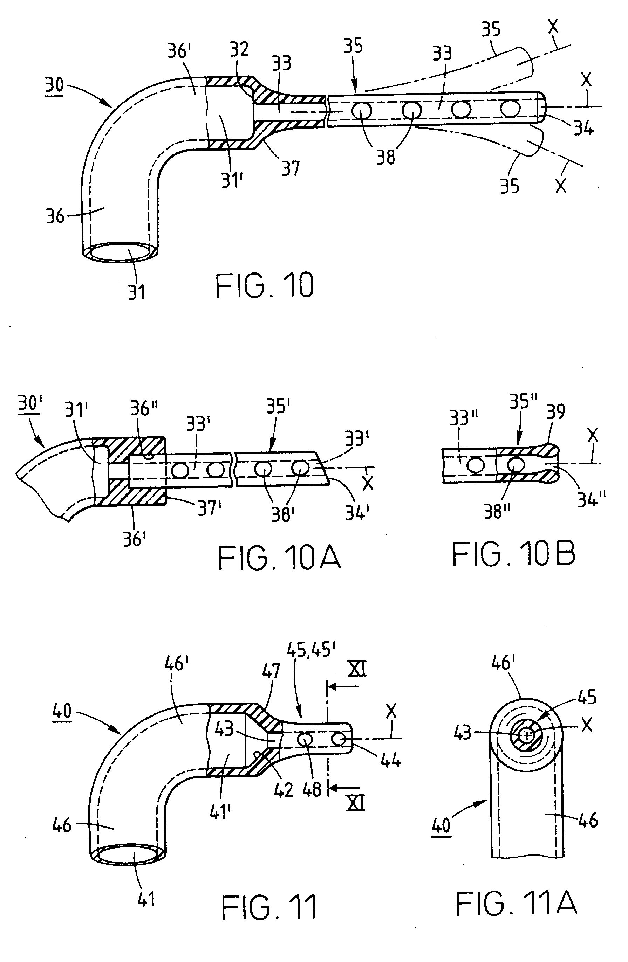

Einzelheiten und weitere zweckmässige Ausgestaltungen der jeweils mit der ersten Zuführleitung 28 in Verbindung stehenden Anschlussteile 30,40 und 50 sowie die daran angeformten beziehungsweise angeordneten Sonden 35,45 und 55 sowie weitere Ausgestaltungen derselben werden nachstehend in Verbindung mit den Figuren 10, 10A, 10B und 11, 11A bis 11D sowie 12, 12A, 12B und 13 im einzelnen beschrieben.

In Fig.6 ist das Teilstück des Auges 15 gemäss Fig.5 dargestellt und man erkennt das Teilstück der Sklera 3 sowie die beiden in Richtung der Kornea 4 aufgeschwenkten oder hochgeklappten Skleralappen 10 und 12, die zweite Ausnehmung 13 mit der seitlichen Auflagefläche 14 der Sklera 3 sowie das freigelegte Teilstück 18 des Schlemmschen Kanals 5. Weiterhin erkennt man eine in den freigelegten Schlemmschen Kanal 5 eingeführte und in der Gesamtheit mit 25' bezeichnete zweite Injektionseinheit. Bei diesem Ausführungsbeispiel ist an die Zuführleitung 28' der schematisch dargestellten Injektionseinheit 25' ein **T**-förmig ausgebildetes und mit zwei gegenüberliegenden Sonden 65 und 65' versehenes Anschlussteil 60 angeordnet.

Bei dem in Fig.6 dargestellten Ausführungsbeispiel besteht die Möglichkeit, dass in einer ersten Phase die eine Sonde 65 zum Injizieren der hochviscosen Natrium-Hyaluronat-Lösung in die eine im Bereich des Teilstücks 18 freigelegte Öffnung 17 des Schlemmschen Kanals 5 eingeführt wird. Nach dem Aufweiten des Schlemmschen Kanals 5 kann das **T**-förmige Anschlussteil 60 mit der Sonde 65 aus dem Schlemmschen Kanal 5 herausgezogen und durch eine geringe seitliche Bewegung in Pfeilrichtung Z' mit der gegenüberliegenden Sonde 65' in die andere Öffnung 17' des Schlemmschen Kanals 5 zum Injizieren des Mediums und Aufweiten des Lumens eingeführt werden. Bei dieser Variante wird das Medium nacheinander in die eine Öffnung 17 und anschliessend in die andere Öffnung 17' eingepresst.

Bei einer nicht dargestellten Variante besteht jedoch auch die Möglichkeit, dass das **T**-förmige Anschlussteil 60 im Bereich des freigelegten Teilstücks 18 zuerst mit der einen Sonde 65 in die eine Öffnung 17 und gleichzeitig durch eine geringe seitliche Verschiebung gemäss Pfeilrichtung Z' mit der zweiten Sonde 65' in die andere gegenüberliegende Öffnung 17' eingeführt wird. Bei dieser Variante besteht folglich die Möglichkeit, dass das Medium mittels der Druckquelle 26' gleichzeitig in die beiden Öffnungen 17 und 17' des freigelegten Schlemmschen Kanals 5 eingepresst werden kann.

Die in Fig.6 dargestellte Injektionseinheit 25' steht über eine daran angeschlossene Zuführleitung 28' mit der Druckquelle 26' in Verbindung. Mittels der beispielsweise manuell oder elektrisch betätigbaren Druckquelle 26' wird das zu injizierende Medium gemäss Pfeilrichtung 27' in das Lumen des Schlemmschen Kanals 5 gepresst. Die in Fig.6 dargestellte Druckquelle 26' ist in nicht näher dargestellter Weise vorzugsweise als Zweikammer-Spritze ausgebildet. Hierdurch besteht die Möglichkeit, dass das Medium entweder mit der einen Sonde 65 oder mit der anderen gegenüberliegenden Sonde 65' beziehungsweise gleichzeitig mit beiden Sonden 65 und 65' in das Lumen des Schlemmschen Kanals 5 eingepresst werden kann.

Nach dem Aufweiten des Schlemmschen Kanals 5 mittels der Injektionseinheit 25 (Fig.5) beziehungsweise der Injektionseinheit 25' (Fig.6) werden diese in nicht dargestellter Weise wieder entfernt.

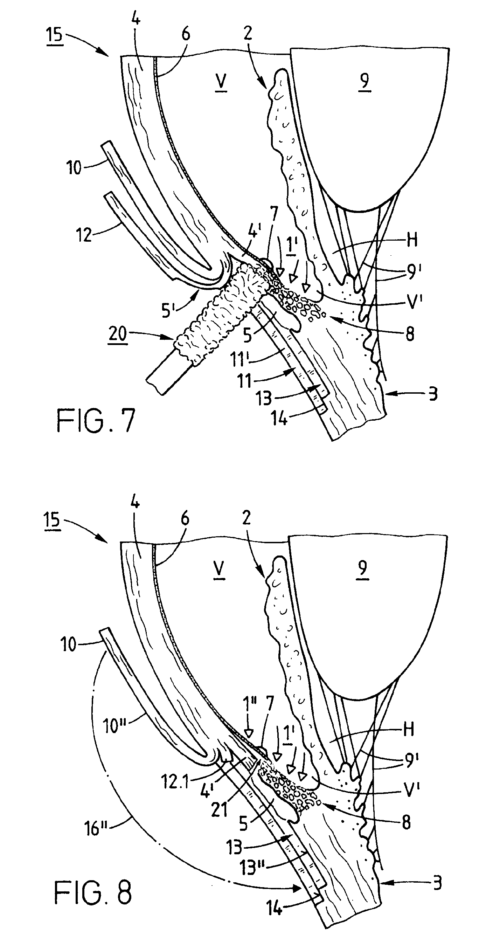

In einer nächsten Phase wird, wie in Fig.7 dargestellt, mittels eines Tupfers 20 oder dergleichen infolge einer geringen Kraft mit dem Tupfer 20 im Bereich der Schwalbeschen Linie die Descemet-Membrane 6 von der Innenseite der Kornea 4 derart gelöst, dass zwischen der Kornea 4 und der Descemet-Membrane 6 ein in Fig.8 in grösserem Massstab und schematisch dargestellter Durchgang 21 entsteht. Der etwa spaltförmige Durchgang 21 erstreckt sich in nicht dargestellter Weise über die gesamte Breite der in Fig.5 und Fig.6 in Ansicht sowie in grösserem Massstab dargestellten zweiten Ausnehmung 13.

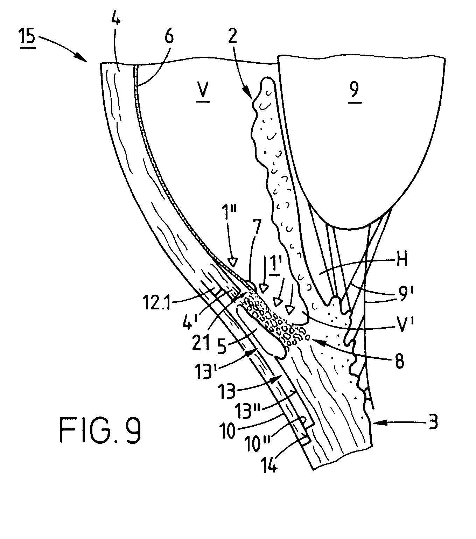

Mit dem spaltförmig ausgebildeten Durchgang 21 wird eine zusätzliche Verbindung zwischen der Vorderkammer V und der zweiten Ausnehmung 13 geschaffen. Das Kammerwasser kann somit zusätzlich zu dem natürlichen Abfluss über das Trabekulargewebe 8 gemäss Pfeilrichtung 1' auch noch über die weitgehend transparente und für das Kammerwasser teildurchlässige Descemet-Membrane 6 gemäss Pfeilrichtung 1" (Fig.8 und Fig.9) und durch den spaltförmigen Durchgang 21 in die mit dem Schlemmschen Kanal 5 in Verbindung stehende Ausnehmung 13 abgeleitet werden. Die dem zweiten Skleralappen 12 entsprechend ausgebildete flächige Ausnehmung 13 bildet im wesentlichen ein Sammelbecken (Reservoir) für das Kammerwasser, welches von diesem in den Schlemmschen Kanal 5 abgeleitet wird.

In einer weiteren Phase wird der zweite Skleralappen 12, vorzugsweise bis auf ein restliches Teilstück 12.1 (Fig.8 und Fig.9), in nicht näher dargestellter Weise mittels eines geeigneten chirurgischen Schneidinstruments abgetrennt. Es besteht jedoch auch die Möglichkeit, dass zuerst der zweite Skleralappen abgetrennt und anschliessend mittels des gegen die Schwalbesche Linie gedrückten Tupfers 20 die Descemet-Membrane 6 von der Innenseite des Teilstücks 4' der Kornea 4 zur Bildung des spaltförmigen Durchgangs 21 gelöst wird.

In einer nächsten Phase wird unter Beibehaltung des spaltförmigen Durchgangs 21 der erste Skleralappen 10 gemäss Pfeilrichtung 16" (Fig.8) herunter geklappt und wie in Fig.9 schematisch dargestellt auf die parabolförmige Auflagefläche 14 gelegt. Anschliessend wird der erste Skleralappen 10 in an sich bekannter, nicht näher dargestellter Weise teilweise mit der Sklera 3 vernäht. Der infolge des abgetrennten zweiten Skleralappens 12 hinter dem ersten Skleralappen 10 entstandene Raum 13' (subscleral space) in Form der flächigen Ausnehmung 13 wird vorzugsweise vor dem vollständigen Vernähen noch mittels einer nicht dargestellten Spritze mit hochviscosem Medium (viscosity sodium hyaluronate) gefüllt. Hierdurch wird verhindert, dass der heruntergeklappte erste Skleralappen 10 mit der Innenseite 10" mit der Innenfläche 13" der Ausnehmung 13 in Kontakt kommt (Fig.9).

An dieser Stelle wird darauf hingewiesen, dass ergänzend zu den vorstehend beschriebenen Ausführungsbeispielen und Varianten die Möglichkeit besteht, dass zur Aufrechterhaltung des verbesserten Kammerwasserabflusses ein Stützmittel aus geeignetem Material in den aufgeweiteten Schlemmschen Kanal 5 beziehungsweise in den spaltförmigen Durchgang 21 und zusätzlich in den subskleralen Raum 13' implantiert wird. Diese Massnahme sowie das speziell ausgebildete Stützmittel sind nicht Gegenstand vorliegener Erfindung und werden deshalb nicht näher beschrieben.

In Fig.10 ist als erstes Ausführungsbeispiel das bogenförmig ausgebildete und mit einem ersten Schenkel 36 sowie einem zweiten Schenkel 36' versehene Anschlussteil 30 in Ansicht und teilweise im Schnitt dargestellt. Das Anschlussteil 30 hat eine Eintrittsöffnung 31, welche in einen im zweiten Schenkel 36' vorgesehenen und durch eine Wand 32 begrenzten Innenraum 31' mündet. An dem zweiten Schenkel 36' des Anschlussteils 30 ist eine röhrchenförmige Sonde 35 angeordnet, welche von einer in axialer Richtung orientierten und mit dem Innenraum 31' des bogenförmigen Anschlussteils 30 in Verbindung stehende Bohrung 33 in Form eines Austrittskanals durchdrungen wird.

Die Sonde 35 ist an dem einen Ende zum Einführen in den Schlemmschen Kanal 5 beispielsweise mit einer bombierten Stirnseite 34 versehen. An dem anderen Ende ist die Sonde 35 mittels eines in Richtung des zweiten Schenkels 36' konisch oder kreisbogenförmig erweiternd ausgebildeten Übergang 37 an dem Anschlussteil 30 angeformt. Der konische oder kreisbogenförmige Übergang 37 gewährleistet beim Einführen in die Öffnung 17,17' des Schlemmschen Kanals 5 (Fig.5) eine abdichtende Anlage, so dass ein Entweichen des injizierten Mediums bei etwaigem Rückstau im Lumen des Schlemmschen Kanals 5 weitgehend verhindert wird.

Bei dem in Fig.10 dargestellten ersten Ausführungsbeispiel ist die am Anschlussteil 30 angeordnete beziehungsweise angeformte Sonde 35 als längliches und flexibles Röhrchen ausgebildet. Die als flexibles Röhrchen ausgebildete Sonde 35 ist wie in Fig.10 schematisch dargestellt, in Bezug auf die Längsachse X in einem räumlichen Bereich hinsichtlich seiner Lage und Orientierung frei beweglich ausgebildet. Die Sonde 35 kann für den Austritt des in den Schlemmschen Kanal 5 (Fig.5) zu injizierenden Mediums zusätzlich mit einer Anzahl in axialer Richtung im Abstand zueinander angeordneten Austrittsöffnungen 38 versehen sein, welche mit der als Austrittskanal ausgebildeten Bohrung 33 in Verbindung stehen.

Bei einer nicht näher dargestellten Variante besteht auch die Möglichkeit, dass die Sonde 35 ausgehend von dem distalen Ende beziehungsweise der Stirnseite 34 in Richtung des Übergangs 37 konisch erweiternd ausgebildet ist.

Das Röhrchen kann sich aufgrund der Flexibilität beim Einführen in den Schlemmschen Kanal 5 (Fig.6) hinsichtlich der Lage und Orientierung an die jeweilige innere Formgebung des Schlemmschen Kanals 5 selbsttätig anpassen. Die Sonde 35 ist vorzugsweise aus einem flexiblen Röhrchen, beispielsweise aus einem transparenten, flexiblen Kunststoffröhrchen hergestellt. Weiterhin kann die Sonde 35 aber auch aus einem flexiblen Metallröhrchen bestehen, welches beispielsweise aus einer Nickel-Titan-Legierung hergestellt ist. Bei den vorstehend erwähnten Beispielen sind die Röhrchen mit derart begrenzter Flexibilität hergestellt, dass ein Abknicken bei einer längeren Ausführungsform der Sonde ausgeschlossen ist.

In Fig.10A ist eine erste Variante der an dem zweiten Schenkel 36' des Anschlussteils 30' angeordneten Sonde 35' dargestellt. Bei dieser Variante ist die röhrchenförmige Sonde 35' in eine entsprechend ausgebildete Ausnehmung 36" des Anschlussteils 30' eingeschoben und durch geeignete Mittel, beispielsweise durch eine Klebverbindung mit dem Anschlussteil 30' fest verbunden. Die röhrchenförmige Sonde 35' steht über eine in axialer Richtung orientierte und als Austrittkanal ausgebildete Bohrung 33' mit dem Innenraum 31' in Verbindung. Weiterhin ist die Sonde 35' für den Austritt des in den Schlemmschen Kanal 5 (Fig.5) zu injizierenden Mediums mit einer Anzahl in axialer Richtung im Abstand zueinander angeordneten Austrittsöffnungen 38' versehen, welche mit der Bohrung 33' in Verbindung stehen. Bei dieser Variante ist die distale Stirnseite als schräg rückwärts geneigte Fläche 34' ausgebildet. Die in Bezug auf den Aussendurchmesser der Sonde 35' abgesetzte kreisringförmige Stirnseite 37' des Anschlussteils 30' gewährleistet beim Einführen in den Schlemmschen Kanal 5 (Fig.5) ebenfalls eine weitgehend abdichtende Anlage an der Öffnung 17 und 17', so dass ein Entweichen des injizierten Mediums bei etwaigem Rückstau im Lumen des Schlemmschen Kanals 5 ebenfalls verhindert wird.

In Fig.10B ist eine zweite Variante der mit der Bohrung 33" und den Austrittsöffnungen 38' versehenen und an einem nicht dargestellten Anschlussteil angeordneten und röhrchenförmig ausgebildeten Sonde 35" dargestellt. Bei dieser Variante ist die distale Stirnseite 34" der Sonde 35" als zirkulärer Wulst 39 ausgebildet.

An dieser Stelle wird darauf hingewiesen, dass die an dem jeweiligen Anschlussteil angeordnete Sonde 35' gemäss Fig.10A sowie die Sonde 35" gemäss Fig.10B analog der vorstehend in Verbindung mit Fig.10 beschriebenen und an dem bogenförmigen Anschlussteil 30 angeordneten Sonde 35 aus einem flexiblen Kunststoffröhrchen oder aus einem flexiblen, beispielsweise aus einer Nickel-Titan-Legierung hergestellten Metallröhrchen mit begrenzter Flexibilität hergestellt werden können.

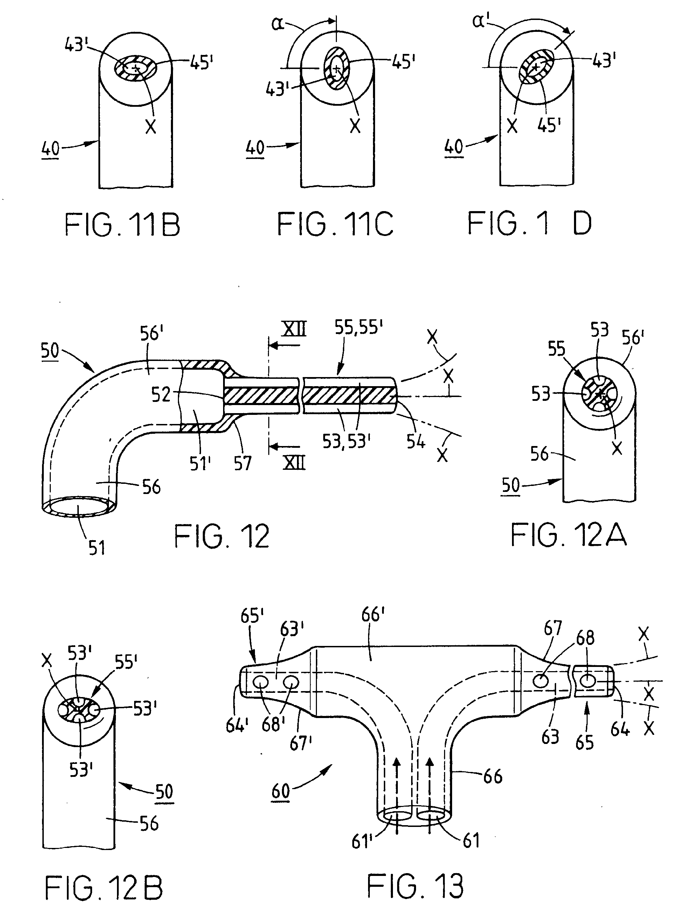

Fig.11 zeigt als zweites Ausführungsbeispiel ein in Ansicht sowie teilweise im Schnitt dargestelltes und mit einer Eintrittsöffnung 41 versehenes Anschlussteil 40. Das bogenförmig ausgebildete Anschlussteil 40 umfasst einen ersten Schenkel 46 sowie einen zweiten Schenkel 46' und ist im wesentlichen analog dem vorstehend in Verbindung mit Fig.10 beschriebenen Anschlussteil 30 ausgebildet. Abweichend davon ist an dem zweiten Schenkel 46' eine relativ kurze und mit einer Längsachse X versehene Sonde 45 oder 45' angeformt, welche von einer in axialer Richtung orientierten und mit dem Innenraum 41' des Anschlussteils 40 in Verbindung stehenden Bohrung 43 in Form eines Austrittskanals durchdrungen wird. Die Sonde 45 kann weiterhin mit einer Anzahl in axialer Richtung im Abstand zueinander angeordneten Austrittsöffnungen 48 versehen sein, welche mit der Bohrung 43 in Verbindung stehen. Zum Einführen in den Schlemmschen Kanal 5 ist die Sonde 45 beispielsweise mit einer bombierten Stirnseite 44 versehen.

An dem anderen Ende ist die Sonde 40 mittels eines in Richtung des zweiten Schenkels 46' konisch oder kreisbogenförmig erweiternd ausgebildeten Übergang 47 an dem Anschlussteil 40 angeformt. Der konische oder kreisbogenförmige Übergang 47 gewährleistet beim Einführen in die Öffnung 17,17' des Schlemmschen Kanals 5 (Fig.5) eine abdichtende Anlage, so dass ein Entweichen des injizierten Mediums bei etwaigem Rückstau im Lumen des Schlemmschen Kanals 5 weitgehend verhindert wird. Bei einer nicht näher dargestellten Variante besteht auch die Möglichkeit, dass die Sonde 45 ausgehend von dem distalen Ende beziehungsweise der Stirnseite 44 in Richtung des Übergangs 47 konisch erweiternd ausgebildet ist.

Der in dem zweiten Schenkel 46' des Anschlussteils 40 vorgesehene Innenraum 41' hat eine trichterförmige Innenwand 42, welche ausgehend von dem Innendurchmesser des Innenraums 41' in Richtung der als Austrittskanal ausgebildeten Bohrung 43 konisch verjüngend ausgebildet ist. Die in Richtung der Bohrung 43 im wesentlichen düsenförmig verjüngend ausgebildete Innenwand 42 bewirkt in vorteilhafterweise einen komprimierten und druckbeschleunigten Austritt des in das Lumen des Schlemmschen Kanals 5 zu injizierenden Mediums.

An dieser Stelle wird darauf hingewiesen, dass die am zweiten Anschlussteil 40 angeordnete Sonde 45 oder 45' in nicht dargestellter Weise auch analog der Sonde 35' gemäss Fig.10A oder analog der Sonde 35" gemäss Fig.10B ausgebildet werden kann. Fig.11A zeigt das entlang der Linie XI-XI im Schnitt dargestellte Anschlussteil 40 mit dem ersten Schenkel 46 sowie die im Profilquerschnitt kreisringförmig ausgebildete und mit der als Austrittskanal ausgebildeten Bohrung 43 versehene Sonde 45.

In Fig.llB ist eine erste Variante der entlang der Linie XI-XI im Schnitt dargestellten sowie am Anschlussteil 40 angeformten Sonde 45' dargestellt. Die Sonde 45' ist hierbei als ein im Profilquerschnitt etwa ellipsenförmig ausgebildetes Röhrchen ausgebildet, welches von einer als Austrittskanal elliptisch ausgebildeten Bohrung 43' durchdrungen wird. In Fig.11c ist die im Profilquerschnitt ellipsenförmige Sonde 45' zur besseren Handhabung und zum besseren Einführen in den Schlemmschen Kanal 5 (5) in Bezug auf die Längsachse X unter rechtem Winkel α und in Fig.llD unter stumpfem Winkel α' an dem zweiten Schenkel 46' des Anschlussteils 40 angeordnet beziehungsweise angeformt.

Fig.12 zeigt als drittes Ausführungsbeispiel ein in Ansicht und teilweise im Schnitt dargestelltes und mit einer Eintrittsöffnung 51 versehenes Anschlussteil 50. Das bogenförmig ausgebildete Anschlussteil 50 umfasst einen ersten Schenkel 56 sowie einen zweiten Schenkel 56' und ist im wesentlichen analog dem vorstehend in Verbindung mit dem in Fig.10 oder Fig.10A beziehungsweise Fig.10B beschriebenen Anschlussteil 30 oder mit dem anhand von Fig.11 oder Fig.11A bis Fig.11D beschriebenen Anschlussteil 40 ausgebildet.

Bei dem dritten Ausführungsbeispiel gemäss Fig.12 ist an dem zweiten Schenkel 56' eine längliche Sonde 55,55' angeformt, welche im Profilquerschnitt kreis- oder ellipsenförmig ausgebildet ist. Die Sonde 55,55' ist abweichend von der Ausführungsform gemäss Fig.10 und Fig.11 am äusseren Umfang mit mindestens einer in axialer Richtung derselben orientierten und als Austrittskanal ausgebildeten Profilnut 53 oder 53' versehen. Die einzelne Profilnut 53 oder 53' steht mit dem durch die Wand 52 begrenzten Innenraum 51' des zweiten Schenkels 56' in Verbindung. Die Sonde 55 beziehungsweise 55' ist analog der vorstehend beschriebenen Sonden 35 und 45 an der Stirnseite 54 beispielsweise bombiert ausgebildet.

An dem anderen Ende ist die Sonde 50 mittels eines in Richtung des zweiten Schenkels 56' konisch oder kreisbogenförmig erweiternd ausgebildeten Übergang 57 an dem Anschlussteil 50 angeformt. Der konische oder kreisbogenförmige Übergang 57 gewährleistet beim Einführen in die Öffnung 17,17' des Schlemmschen Kanals 5 (Fig.5) eine abdichtende Anlage, so dass ein Entweichen des injizierten Mediums bei etwaigem Rückstau im Lumen des Schlemmschen Kanals 5 weitgehend verhindert wird.

Die an dem Anschlussteil 50 angeordnete Sonde 55 oder 55' gemäss Fig.12 kann ebenfalls analog der vorstehend in Verbindung mit Fig.10 beschriebenen und an dem bogenförmigen Anschlussteil 30 angeordneten Sonde 35 aus einem flexiblen Kunststoffröhrchen mit begrenzter Flexibilität hergestellt werden. Die Sonde 55,55' ist in Bezug auf die Längsachse X in einem räumlichen Bereich hinsichtlich ihrer Lage und Orientierung analog Fig.10 frei beweglich ausgebildet.

Die beiden Figuren 12A und 12B zeigen jeweils das Anschlussteil 50 mit der entlang der Linie XII-XII im Schnitt dargestellten Sonde 55 beziehungsweise 55'. Bei dem in Fig.12A dargestellten Anschlussteil 50 ist die Sonde 55 im Profilquerschnitt kreisförmig ausgebildet. Am äusseren Umfang der Sonde 55 sind beispielsweise mehrere verteilt zueinander und mit dem Innenraum 51' des Anschlussteils 50 in Verbindung stehende und jeweils als Austrittskanal ausgebildete Profilnuten 53 angeordnet. Bei dem in Fig.12B dargestellten Anschlussteil 50 ist die angeformte Sonde 55' im Profilquerschnitt elliptisch ausgebildet. Am äusseren Umfang der elliptischen Sonde 55' sind ebenfalls mehrere verteilt zueinander und mit dem Innenraum 51' des Anschlussteils 50 in Verbindung stehende Profilnuten 53' angeordnet.

Die vorstehend in Verbindung mit den Figuren 10 bis 12 beschriebenen Anschlussteile 30,40 oder 50 mit den jeweils daran angeordneten beziehungsweise angeformten Sonden 35,45,45' oder 55,55' sind derart ausgebildet, dass das Medium nacheinander entweder in die eine oder in die andere gegenüberliegende Öffnung bzw. in das Lumen des chirurgisch freigelegten Schlemmschen Kanals 5 injizierbar ist (Fig.6).

Fig.13 zeigt als drittes Ausführungsbeispiel das in Ansicht dargestellte Anschlussteil 60 für die in Fig.6 schematisch dargestellte Injektionseinheit 25'. Das im Profilquerschnitt etwa **T**-förmig ausgebildete Anschlussteil 60 hat an dem oberen Teilstück 66' zwei gegenüberliegend zueinander angeordnete und jeweils von einer als Austrittskanal ausgebildeten Bohrung 63 beziehungsweise 63' durchdrungene Sonden 65 und 65'. Weiterhin ist an dem oberen Teilstück 66' ein quer dazu angeordneter und mit zwei getrennten Eintrittsöffnungen 61,61' versehener Schenkel 66 angeformt. Die Eintrittsöffnungen 61 und 61' stehen mit den in den Sonden 65 und 65' vorgesehenen Bohrungen 63 und 63' in Verbindung. Die beiden Sonden 65 und 65' können hinsichtlich der Länge, wie in Fig.6 schematisch dargestellt, auch unterschiedlich ausgebildet sein.

Die beiden seitlich an dem Teilstück 66' angeformten und mit den als Austrittskanal ausgebildeten Bohrungen 63 und 63' versehenen Sonden 65 und 65' können analog der Sonde 35 (Fig.10) jeweils als flexibles Röhrchen ausgebildet sein, welches beim Einführen in die Öffnung des Schlemmschen Kanals 5 in Bezug auf die Längsachse X hinsichtlich der Lage und Orientierung frei beweglich ist und sich an die Formgebung des Lumens anpasst. Die beiden Sonden 65 und 65' können auch im Profilquerschnitt entsprechend der Variante gemäss Fig.llA und 11B oder aber entsprechend der Variante gemäss Fig.12A und 12B ausgebildet werden.

Die Sonde 65 beziehungsweise 65' ist analog der vorstehend beschriebenen Sonden 35 und 45 an der Stirnseite 64 und 64' beispielsweise bombiert ausgebildet. An dem anderen Ende sind die beiden Sonden 65 und 65' jeweils mittels eines in Richtung des oberen Teilstücks 66' konisch oder kreisbogenförmig erweiternd ausgebildeten Übergangs 67 und 67' an dem **T**-förmig ausgebildeten Anschlussteil 60 angeformt. Der konische oder kreisbogenförmige Übergang 67 und 67' gewährleistet jeweils beim Einführen in die Öffnung 17,17' des Schlemmschen Kanals 5 (Fig.5) eine abdichtende Anlage, so dass ein Entweichen des injizierten Mediums bei etwaigem Rückstau im Lumen des Schlemmschen Kanals 5 weitgehend verhindert wird.

## Patentansprüche

1. Verfahren zum Verbessern des Kammerwasserabflusses in einem Auge (15), bei welchem das abgesonderte Kammerwasser im Bereich des Kammerwinkels (V') der Vorderkammer (V) über das Trabekulargewebe (8) in den Schlemmschen Kanal (5) und anschliessend über das natürliche Kanalsystem abgeleitet wird, **gekennzeichnet durch** einen ersten lamellaren Einschnitt an der Oberfläche der Sklera (3) mit in Richtung der Kornea (4) aufgeschwenktem ersten Skleralappen (10) und analog ausgebildeter erster Ausnehmung (11), einen innerhalb der ersten Ausnehmung (11) angeordneten zweiten lamellaren Einschnitt mit in Richtung des ersten Skleralappens (10) aufgeschwenktem zweiten Skleralappen (12) und analog ausgebildeter zweiter Ausnehmung (13) sowie ein im Bereich der zweiten Ausnehmung (13) freigelegtes Teilstück (18) des Schlemmschen Kanals (5) mit zwei gegenüberliegenden Öffnungen (17,17') zum Injizieren eines den Schlemmschen Kanal (5) dehnenden Mediums, wobei der erste Skleralappen (10) nach dem Abtrennen des zweiten Skleralappens (12) auf eine analog dem zweiten Einschnitt ausgebildete Auflagefläche (14) gelegt wird und der dadurch gebildete subsklerale Raum (13') vor dem vollständigen Verschliessen mit einem viskosen Medium gefüllt wird.

2. Verfahren nach Anspruch 1, **dadurch gekennzeichnet,** dass zur Bildung eines die kammerwasserdurchlässige Descemet-Membrane (6) mit der zweiten Ausnehmung (13) beziehungsweise mit dem subskleralen Raum (13') verbindenden Spalts (21) die Descemet-Membrane (6) durch eine geringe Anpresskraft im Bereich der Schwalbeschen Linie (7) von der Kornea (4) gelöst wird.

3. Verfahren nach den Ansprüchen 1 und 2, **dadurch gekennzeichnet,** dass der zweite Skleralappen (12) vor oder nach dem Injizieren des Mediums in den Schlemmschen Kanal (5) beziehungsweise vor oder nach dem Lösen der Descemet-Membrane (6) von der Kornea (4) im Bereich der zweiten Ausnehmung (13) von der Kornea (4) abgetrennt wird.

4. Verfahren nach Anspruch 1, **dadurch gekennzeichnet**, dass das viskose Medium komprimiert und druckbeschleunigt in das Lumen des Schlemmschen Kanals (5) injiziert wird.

5. Vorrichtung zum Dehnen des chirurgisch freigelegten Schlemmschen Kanals (5) in einem Auge (15) infolge des lokalen Druckaufbaus mittels eines injizierten viskosen Mediums, **gekennzeichnet durch** eine in den Schlemmschen Kanal (5) einführbare und für das zu injizierende Medium mit mindestens einem Austrittskanal (33,33',33";43,43';53,53';63,63') versehene Sonde (35,35',35";45,45';55,55';65,65'), welche eine in axialer Richtung orientierte und mindestens dem zweifachen Durchmesser entsprechende Länge aufweist.

6. Vorrichtung nach Anspruch 5, **dadurch gekennzeichnet,** dass die Sonde (35,35',35";45,45') im Profilquerschnitt als kreisringförmiges oder elliptisches Röhrchen ausgebildet sowie an einem bogenförmigen Anschlussteil (30;40) angeordnet und über eine in axialer Richtung orientierte und als Austrittskanal ausgebildete Bohrung (33,33',33";43,43') mit dem Innenraum (31';41') des mit einer Druckquelle (26) in Verbindung stehenden Anschlussteils (30;40) verbunden ist.

7. Vorrichtung nach Anspruch 5, **dadurch gekennzeichnet,** dass die Sonde (55,55') im Profilquerschnitt als kreisförmiger oder elliptischer Körper ausgebildet und an einem bogenförmigen Anschlussteil (50) angeordnet ist, wobei die Sonde (55,55') über mindestens eine am äusseren Umfang angeordnete sowie in axialer Richtung orientierte und als Austrittskanal ausgebildete Profilnut (53,53') mit dem Innenraum (51') des Anschlussteils (50) verbunden ist.

8. Vorrichtung nach Anspruch 5, **gekennzeichnet durch** ein im Profilquerschnitt **T**-förmig ausgebildetes Anschlussteil (60) mit zwei an dem oberen Teilstück (66') gegenüberliegend zueinander angeordneten und im Profilquerschnitt als kreisringförmiges oder elliptisches Röhrchen ausgebildeten Sonden (65,65'), welche über darin angeordnete und als Austrittskanal ausgebildete Bohrungen (63,63') mit an dem angeformten Schenkel (66) angeordneten Eintrittsöffnungen (61,61') verbunden sind.

9. Vorrichtung nach Anspruch 8, **dadurch gekennzeichnet,** dass das **T**-förmige Anschlussteil (60) mit dem Schenkel (66) derart an eine mit der Druckquelle (26) in Verbindung stehende Zuführleitung (28') angeschlossen ist, dass den beiden als Austrittskanal ausgebildeten Bohrungen (63,63') das zu injizierende Medium gleichzeitig oder nacheinander zuführbar ist.

10. Vorrichtung nach Anspruch 5, **dadurch gekennzeichnet,** dass die Sonde (35,35',35";45,45';55,55';65,65') eine dem mehrfachen Durchmesser entsprechende Länge aufweist und hinsichtlich der Lage und Orientierung in räumlichem Bereich frei beweglich ist.

11. Vorrichtung nach Anspruch 5, **dadurch gekennzeichnet,** dass die Sonde (35,35',35";45,45';55,55';65,65') ausgehend von dem als Stirnseite (34;44;54;55,55') ausgebildeten distalen Ende in Richtung des mit dem Anschlussteil (30,30';40; 50;60) verbundenen proximalen Endes konisch erweiternd ausgebildet ist.

12. Vorrichtung nach Anspruch 5, **dadurch gekennzeichnet,** dass die Sonde (35,35',35";45,45';55,55';65,65') an dem proximalen Ende mit einem konisch oder kreisbogenförmig erweiternd ausgebildeten Übergang (37;47;57;67,67') an dem Anschlussteil (30,30';40;50;60) angeordnet ist.

13. Vorrichtung nach Anspruch 5, **dadurch gekennzeichnet,** dass die Sonde (35,35',35";45,45';55,55';65,65') an dem jeweiligen Anschlussteil (30,30';40;50;60) angeformt oder in eine entsprechend der Sonde ausgebildete Ausnehmung eingesteckt und befestigt ist.

14. Vorrichtung nach Anspruch 5, **dadurch gekennzeichnet,** dass die Sonde (35,35',35";45,45';55,55';65,65') aus flexiblem Kunststoff, beispielsweise aus transparentem Kunststoff hergestellt ist.

15. Vorrichtung nach Anspruch 5, **dadurch gekennzeichnet,** dass die Sonde (35,35',35";45,45';55,55';65,65') aus einem flexiblem Metallröhrchen hergestellt ist.

16. Vorrichtung nach Anspruch 15, **dadurch gekennzeichnet,** dass die Sonde (35,35',35";45,45';55,55';65,65') aus einer Nickel-Titan-Legierung hergestellt ist.
